# FASCICULE DE BREVET EUROPEEN

(11) **EP 3 644 135 B1**
(45) Date de publication et mention de la délivrance du brevet: **23.04.2025**
(21) Numéro de dépôt: 18202778.9
(22) Date de dépôt: 26.10.2018
(51) Int. Cl.: G04G 21/02, A61B 5/00, A61B 5/024

(54) **PROCÉDÉ DE DIFFUSION PAR UNE MONTRE D'UN MESSAGE INFORMATIF RELATIF À UNE ÉVALUATION DE LA QUALITÉ DE VIE D'UN PORTEUR DE LADITE MONTRE**
VERFAHREN ZUR VERBREITUNG ÜBER EINE ARMBANDUHR EINER INFORMATION ÜBER EINE BEWERTUNG DER SCHLAFQUALITÄT EINES TRÄGERS DIESER ARMBANDUHR
METHOD FOR BROADCASTING BY A WATCH OF AN INFORMATION MESSAGE RELATING TO AN EVALUATION OF THE QUALITY OF LIFE OF A WEARER OF THE WATCH

(43) Date de publication de la demande: 29.04.2020
(73) Titulaire: Tissot S.A., 2400 Le Locle (CH)
(72) Inventeur: FRANZI, Edoardo, 1400 Cheseaux-Noréaz (CH); DUNBAR, Andrea, 2072 St-Blaise (CH); TÜRETKEN, Engin, 1024 Ecublens (CH); MOSER, Virginie, 2517 Diesse (CH); STADELMANN, Patrick, 2017 Boudry (CH); ZHOU, Lingchuan, 2074 Marin-Epagnier (CH)
(74) Mandataire: ICB SA

(56) Documents cités:
- WO-A2-2007/107900
- WO-A2-2011/109716
- US-A1- 2016 246 259

## Description

### Domaine technique

La présente invention concerne un procédé de diffusion par une montre d'un message informatif relatif à une évaluation de la qualité de vie d'un porteur de ladite montre et un système mettant en œuvre un tel procédé.

L'invention concerne également une montre comprenant un tel système ainsi qu'un programme d'ordinateur.

### Art antérieur

La variation de la qualité de vie d'un individu à un impact sur différents états de ce dernier tels que l'état physique, l'état psychologique ou encore l'état somatique. En pareil contexte, on comprend alors qu'il est important de pouvoir évaluer cette qualité de vie d'un individu afin notamment de pouvoir le cas échéant l'améliorer.

Pour ce faire, on connait dans l'état de la technique des procédés prévoyant de réaliser une évaluation de la qualité de vie par la mise en œuvre d'un traitement de données de mesures provenant généralement de capteurs de suivi d'activité ou encore de capteurs physiologiques.

On connait aussi dans l'état de la technique le document WO2007/107900A2 qui décrit un procédé d'affichage d'un degré de relaxation d'un porteur d'une montre ainsi que le document US2016/246259A1 qui porte sur un procédé permettant de rappeler automatiquement à un utilisateur de dormir. On connait également le document WO2011109716A2 qui décrit un procédé de surveillance permanente d'un ou de plusieurs paramètres physiologiques d'un sujet.

Toutefois un des inconvénients majeurs de tels procédés est lié au fait que l'évaluation de la qualité de vie proposée est souvent imprécise voire erronée du fait qu'elle soit réalisée à partir de données de mesure parfois difficiles à obtenir et qui ne sont pas toujours en lien direct avec des conditions liées à la qualité de vie.

On comprend qu'il existe un besoin de trouver une solution alternative, notamment qui ne présente pas les inconvénients de l'art antérieur.

### Résumé de l'invention

Un but de la présente invention est par conséquent de proposer un procédé de diffusion par une montre d'un message informatif relatif à une évaluation de la qualité de vie qui soit fiable et simple de mise en œuvre.

Dans ce dessein, l'invention porte sur un procédé de diffusion par une montre d'un message informatif relatif à une évaluation de la qualité de vie d'un porteur de ladite montre, selon la revendication 1.

Dans d'autres modes de réalisation :
- un premier critère de sélection prévoit qu'au moins une mesure environnementale des données descriptives de chaque épisode événementiel relatif à chaque type d'événement environnemental est comparée à un seuil de référence de perturbation de la qualité de vie ;
- un deuxième critère de sélection consiste à comparer une durée de chaque épisode événementiel relatif à chaque type d'événement environnemental à un seuil de référence de durée de perturbation de la qualité de vie ;
- l'étape d'identification comprend une sous-étape de génération d'un indicateur de perturbation de la qualité de vie pour chaque type d'événement environnemental perturbateur de la qualité de vie identifié ;
- la sous-étape de génération comprend une phase de calcul de l'indicateur de perturbation de la qualité de vie pour chaque type d'événement environnemental perturbateur de la qualité de vie identifié à partir des caractéristiques descriptives suivantes :
   ▪ une valeur moyenne du ou des mesures environnementales comprises dans les données descriptives des épisodes événementiels sélectionnés relatifs à ce type d'événement environnemental ;
   ▪ une durée totale du déroulement de ce type d'événement environnemental ;
   ▪ le nombre d'épisodes événementiels sélectionnés de ce type d'événement environnemental ;
   ▪ des heures de début et de fin des épisodes événementiels sélectionnés de ce type d'événement environnemental ;
- la période déterminée est définie automatiquement par l'unité de traitement en étant initié dès lors qu'un premier épisode événementiel d'un type d'événement environnemental est identifié, et achevée lorsqu'une durée d'exposition du porteur à au moins un type d'événement environnemental considéré comme perturbateur de la qualité de vie est supérieure à un seuil de durée de référence.

L'invention porte aussi sur un système de diffusion par une montre d'un message informatif relatif à une évaluation de la qualité de vie d'un porteur de ladite montre mettant en œuvre ce procédé, le système comprenant les éléments suivants reliés entre eux : une unité de traitement, au moins un capteur environnemental et une interface de diffusion d'une information visuelle et/ou sonore.

L'invention porte également sur une montre comportant un tel système.

Avantageusement, la montre est une montre mécanique connectée.

L'invention porte en outre sur un programme d'ordinateur comprenant des instructions de code de programme pour l'exécution des étapes du procédé lorsque ledit programme est exécuté par une unité de traitement.

### Brève description des figures

D'autres particularités et avantages ressortiront clairement de la description qui en est faite ci-après, à titre indicatif et nullement limitatif, en référence aux figures annexées, dans lesquelles :
- la figure 1 est une représentation schématique d'une montre comprenant un système de diffusion d'un message informatif relatif à une évaluation de la qualité de vie du porteur de ladite montre, selon un mode de réalisation de l'invention, et
- la figure 2 est un logigramme relatif à un procédé de diffusion de ce message informatif, selon le mode de réalisation de l'invention.

### Description détaillée de l'invention

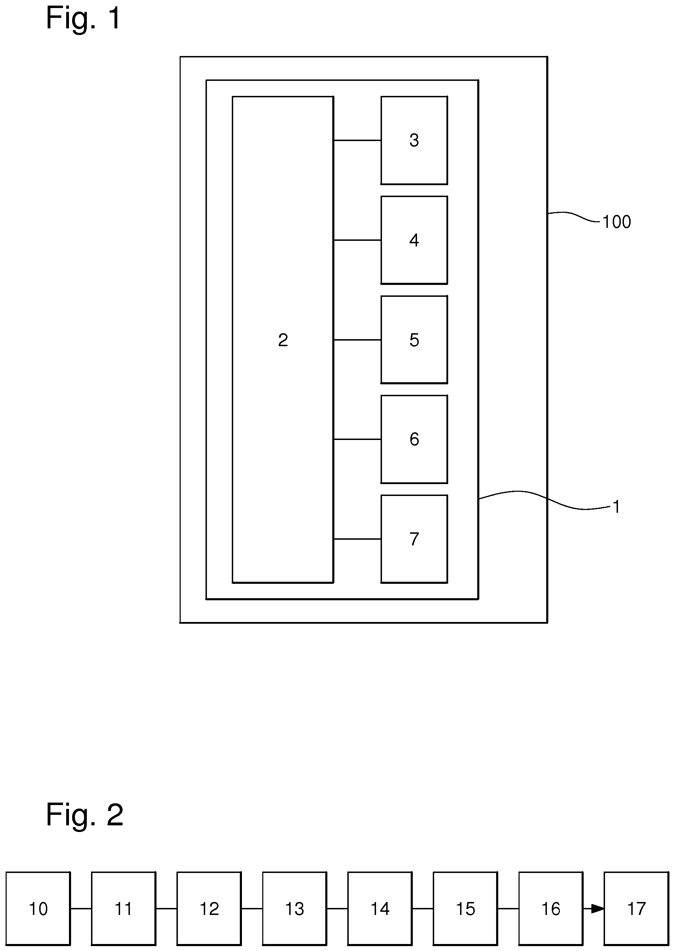

Sur la figure 1 est représentée une montre 100 comprenant le système de diffusion 1 par cette montre 100 d'un message informatif relatif à une évaluation de la qualité de vie d'un porteur de ladite montre 100. Un tel système 1 est compris dans la montre 100 qui est de préférence une montre 100 mécanique connecté à affichage hybride. Ce système 1 comprend plus précisément de manière non limitative et/ou non exhaustive :
- - une unité de traitement 2 comportant des ressources matérielles et logicielles en particulier au moins un processeur coopérant avec des éléments de mémoire ;
- - une interface de diffusion d'une information visuelle 3 telle qu'un cadran d'affichage hybride pourvu d'une première composante d'affichage analogique et d'une deuxième composante d'affichage numérique et/ou alphanumérique ;
- - une interface de diffusion d'une information sonore 4 telle qu'un haut-parleur ;
- - une interface de communication ;
- - au moins un capteur environnemental 5 ;
- - au moins un capteur comportemental 6, et
- - au moins un capteur physiologique 7.

Dans ce système 1, l'unité de traitement 2 est reliée entre autres aux interfaces de diffusion d'une information visuelle et sonore et aux capteurs environnemental 5, comportemental 6, et physiologique 7.

Le système 1 dans cette montre 100 est apte à évaluer la qualité de vie du porteur de préférence uniquement à partir d'au moins un type d'événement environnemental relevé durant une période déterminée. Chaque type d'événement relevé est quantifié à partir d'au moins un épisode événementiel qui lui est propre et qui se déroule pendant la période déterminée. Chaque épisode événementiel est caractérisé par des données descriptives comprenant notamment une ou plusieurs mesures d'un paramètre environnemental. Ce paramètre environnemental est une grandeur relative à une caractéristique de l'environnement dans lequel est présent la montre 100 et son porteur. Un tel paramètre est de manière non limitative et/ou non exhaustive relatif à : une température, une hygrométrie, un niveau de bruit ambiant, la fréquence d'un son continu, une pression atmosphérique, un éclairement, la qualité de l'air, la quantité de soleil, le manque de lumière, etc...

A titre d'exemple, lorsque le paramètre correspond à un « niveau de bruit », un épisode événementiel est défini par les données descriptives suivantes :
- une ou plusieurs mesures de ce niveau de bruit sont déterminées par au moins un capteur environnemental idoine 5 et transmises à l'unité de traitement 2 ;
- la durée du déroulement de l'épisode événementiel qui est calculée par l'unité de traitement 2 et est par exemple de 10 minutes, et
- l'heure de début 10 heures du matin et l'heure de fin 10 heures 10 minutes sont déterminées par l'unité de traitement 2.

On comprend qu'à ce stade cet événement environnemental est lié à un « niveau de bruit », et que pour ce type d'événement plusieurs épisodes avec des données descriptives différentes peuvent alors être estimés durant la période déterminée. Par la suite, selon le procédé décrit plus après, si ce type d'événement environnemental est considéré comme étant perturbateur de la qualité de vie du porteur il devient alors une « nuisance sonore ».

Dans ce contexte, les capteurs environnementaux 5 sont spécifiquement adaptés pour mesurer ces paramètres environnementaux. Ainsi que nous le verrons par la suite les autres capteurs comportementaux 6 et physiologiques 7 participent à la réalisation de mesures qui peuvent être utilisées de manière optionnelle par l'unité de traitement 2 dans le cadre de l'évaluation de la qualité de vie du porteur. Les capteurs comportementaux 6 sont aptes à mesurer tous types de caractéristiques comportementales du porteur de la montre 100 comme par exemple les mouvements ou les gestes réalisés par ce dernier pendant la période déterminée. Pour ce faire, ces capteurs comportementaux 6 peuvent comprendre un ou plusieurs capteurs inertiels de type accéléromètre, gyroscope ou gyromètre multiaxes miniature tels que des capteurs multiaxes fabriqués en technologie MEMS, capables de détecter des vitesses angulaires et des accélérations linéaires selon plusieurs axes associant accéléromètres et/ou gyroscopes. S'agissant des capteurs physiologiques 7, ils sont aptes à mesurer les paramètres relatifs au fonctionnement d'un organisme du porteur tels que, par exemple, le pouls, la saturation du sang en oxygène, l'impédance de la peau, la tension artérielle, le rythme respiratoire, l'arythmie respiratoire, la température cutanée, le taux de sudation, la saturation du sang en oxygène ou le débit sanguin.

Un tel système 1 de la montre 100 est apte à mettre en œuvre un procédé de diffusion par la montre 100 de ce message informatif représenté sur la figure 2.

Ce procédé comprend une étape d'enregistrement 10 par l'unité de traitement 2 de données descriptives d'au moins un épisode événementiel d'au moins un type d'événement environnemental relevé durant une période déterminée. Lors de cette étape 10, dès la détection du début de la période déterminée jusqu'à la fin de cette période, les données descriptives d'un ou plusieurs épisodes événementiels d'au moins un type d'événement environnemental sont archivées dans les éléments de mémoire de l'unité de traitement 2 de la montre 100. Ces données descriptives de chaque épisode événementiel comprennent de manière non exhaustive et non limitative :
- une ou plusieurs mesures environnementales déterminées par les capteurs 5 correspondants et transmises à l'unité de traitement 2 ;
- la durée du déroulement de l'épisode événementiel qui est calculée par l'unité de traitement 2, et
- les heures de début et de fin de l'épisode qui sont déterminées par l'unité de traitement 2.

On notera que la création d'un épisode est liée à l'identification par l'unité de traitement 2 d'une variation d'un paramètre environnemental relatif à un type d'événement environnemental correspondant.

Dans ce contexte, la période déterminée peut être définie automatiquement par l'unité de traitement en étant initié dès lors qu'un premier épisode d'un type d'événement environnemental est identifié, et achevée lorsque la durée d'exposition du porteur à au moins un type d'événement environnemental considéré comme perturbateur de la qualité de vie est supérieure à un seuil de durée de référence.

Alternativement, la période déterminée peut être prédéfinie pour qu'une évaluation de la qualité de vie soit réalisée à intervalle régulier par exemple toute les 4 heures. Le début de cette période peut alors être initié par le porteur de la montre par l'activation d'un élément de commande par exemple un bouton poussoir de cette montre.

Le procédé comprend ensuite une étape d'identification 11 d'au moins un type d'événement environnemental perturbateur de la qualité de vie du porteur à partir d'un traitement desdites données descriptives. Pour ce faire, cette étape 11 comprend une sous-étape de sélection 12 d'un ou plusieurs épisodes événementiels relatifs à chaque type d'événement environnemental relevé durant la période déterminée à partir d'au moins un critère de sélection. Selon un premier critère de sélection, au moins une mesure environnementale des données descriptives de chaque épisode événementiel relatif à chaque type d'événement environnemental, est comparée à un seuil de référence de perturbation de la qualité de vie. Si ladite au moins une mesure est supérieure au seuil de référence de perturbation de la qualité de vie, alors l'épisode peut alors être sélectionné ou présélectionné lorsque les conditions de cette sélection sont liées à la vérification d'un deuxième critère de sélection. Ce deuxième critère de sélection consiste à comparer la durée de chaque épisode relatif à chaque type d'événement environnemental à un seuil de référence de durée de perturbation de la qualité de vie. Dans ce contexte, si la durée de l'épisode est supérieure au seuil de référence de durée de perturbation de la qualité de vie, alors l'épisode est sélectionné.

L'étape d'identification 11 comprend ensuite une sous-étape de détection 13 d'un ou plusieurs types d'événements environnementaux perturbateurs de la qualité de vie du porteur de la montre 100 et ce, à partir des épisodes événementiels qui ont été sélectionnées. Cette sous-étape peut prévoir pour un type d'événement environnemental donné que le nombre d'épisode de cet événement doit être supérieur à un seuil de référence pour être détecté.

Par la suite, l'étape d'indentification 11 comprend une sous-étape de génération 14 d'un indicateur de perturbation de la qualité de vie pour chaque type d'événement environnemental perturbateur de la qualité de vie détecté/identifié. Cette sous-étape 14 comprend une phase 15 de calcul de l'indicateur de perturbation de la qualité de vie pour chaque type d'événement environnemental perturbateur de la qualité de vie identifié à partir de caractéristiques descriptives. Ces caractéristiques descriptives de chaque type d'événement environnemental perturbateur de la qualité de vie identifié sont les suivantes :
- une valeur moyenne du ou des mesures environnementales comprises dans les données descriptives des épisodes événementiels sélectionnés ;
- la durée totale du déroulement de ce type d'événement environnemental perturbateur de la qualité de vie durant la période déterminée de la montre 100 qui est égale à la somme des durées des épisodes événementiels sélectionnées ;
- le nombre d'épisodes événementiels sélectionnés ;
- les heures de début et de fin des épisodes événementiels sélectionnés qui permettent d'évaluer leur effet/impact sur la perturbation des cycles de la qualité de vie. En effet, la période déterminée comprend une succession de cycles avec des phases de la qualité de vie lent, surtout en début de nuit, qui permet à au corps de récupérer physiquement, et de la qualité de vie paradoxal qui correspond au moment où le porteur rêve et où il évacue sa tension nerveuse.

On notera que le calcul de l'indicateur de perturbation de la qualité de vie est réalisé en fonction d'une ou de plusieurs de ces caractéristiques descriptives.

En outre, une telle étape d'identification 11 peut être mise en œuvre dès la fin de la période déterminée ou de manière simultanée à l'étape d'enregistrement 10 c'est-à-dire dès le début de cette période déterminée.

Le procédé comprend ensuite une étape d'estimation 16 d'un indice d'évaluation de la qualité de vie en fonction de l'indicateur de perturbation de la qualité de vie relatif à chaque type d'événement environnemental perturbateur de la qualité de vie identifié. Lors de cette étape 16, l'unité de traitement 2 détermine l'indice d'évaluation de la qualité de vie en calculant une valeur moyenne du ou des indicateurs de perturbation de la qualité de vie obtenus lors de la sous-étape de génération 14. Lors de ce calcul des coefficients peuvent être appliquées à certains indicateurs en fonction de la nature du type de l'événement environnemental perturbateur de la qualité de vie auxquels ils se rapportent. En effet, certains types d'événements peuvent avoir un impact plus important que d'autres sur la perturbation de la qualité de vie.

On notera de manière optionnelle que des données de mesure physiologique et/ou comportementale réalisées durant les épisodes événementiels peuvent également être prises en compte dans le calcul de cet indice.

Ensuite, le procédé comprend une étape de conception 17 du message informatif comprenant l'indice d'évaluation estimé en prévision de sa diffusion à destination du porteur. Un tel message informatif peut être un message sonore ou encore un message visuel comprenant une représentation graphique bidimensionnelle ou tridimensionnelle comportant l'indice. Ce message peut comprendre en plus de l'indice d'évaluation de la qualité de vie, une recommandation à l'attention du porteur de la montre 100 relative à une attitude (ou comportement) à adopter pour améliorer cette qualité de vie qui est définie en fonction de la valeur de cet indice et donc du niveau de la qualité de vie évaluée.

On notera que le procédé peut aussi prévoir une étape durant laquelle, le porteur peut faire part de son évaluation de la qualité de vie sur la journée en choisissant dans un menu contextuel affiché dans une deuxième composante d'affichage du cadran, un critère relatif à la qualification de cette qualité de vie sur la journée parmi une liste de critères comprenant par exemple les mentions suivantes : une bonne journée, une journée reposante, une journée pénible, etc...Cette étape prévoit ensuite un archivage de cette évaluation par le porteur de la montre sous la forme de données dont le traitement par l'unité de traitement peut permettre d'améliorer la précision de l'évaluation de la qualité de vie par les présents procédé et système.

L'invention porte aussi sur un programme d'ordinateur comprenant des instructions de code de programme pour l'exécution des étapes 10 à 17 de ce procédé lorsque ledit programme est exécuté par l'unité de traitement 2 de la montre 100.

## Revendications

1. Procédé de diffusion par une montre (100) d'un message informatif relatif à une évaluation de la qualité de vie d'un porteur de ladite montre (100), **caractérisé en ce que** le procédé comporte les étapes suivantes :
- enregistrement (10) par l'unité de traitement (2) de données descriptives d'au moins un épisode événementiel d'au moins un type d'événement environnemental relevé durant une période déterminée ;
- identification (11) d'au moins un type d'événement environnemental perturbateur de la qualité de vie du porteur à partir d'un traitement desdites données descriptives ;
- estimation (16) d'un indice d'évaluation de la qualité de vie du porteur en fonction d'un indicateur de perturbation de la qualité de vie relatif à chaque type d'événement environnemental perturbateur de la qualité de vie identifié, et
- conception (17) du message informatif comprenant l'indice d'évaluation estimé en prévision de sa diffusion à destination du porteur, et **en ce que** l'étape d'identification (11) comprend une sous-étape de sélection (12) d'un ou de plusieurs épisodes événementiels relatifs à chaque type d'événement environnemental perturbateur de la qualité de vie du porteur qui a été relevé durant la période déterminée à partir d'au moins un critère de sélection et **en ce que** chaque épisode événementiel sélectionné a une mesure environnementale correspondante qui est supérieure à un seuil de référence de perturbation de la qualité de vie et a une mesure de la durée correspondante qui est supérieure à un seuil de durée de référence.

2. Procédé selon la revendication précédente, **caractérisé en ce qu'**un premier critère de sélection prévoit qu'au moins une mesure environnementale des données descriptives de chaque épisode événementiel relatif à chaque type d'événement environnemental est comparée à un seuil de référence de perturbation de la qualité de vie.

3. Procédé selon l'une quelconque des revendications 1 et 2, **caractérisé en ce qu'**un deuxième critère de sélection consiste à comparer une durée de chaque épisode événementiel relatif à chaque type d'événement environnemental à un seuil de référence de durée de perturbation de la qualité de vie.

4. Procédé selon l'une quelconque des revendications précédentes, **caractérisé en ce que** l'étape d'identification (11) comprend une sous-étape de génération (14) d'un indicateur de perturbation de la qualité de vie pour chaque type d'événement environnemental perturbateur de la qualité de vie identifié.

5. Procédé selon la revendication précédente, **caractérisé en ce que** la sous-étape de génération (14) comprend une phase (15) de calcul de l'indicateur de perturbation de la qualité de vie pour chaque type d'événement environnemental perturbateur de la qualité de vie identifié à partir des caractéristiques descriptives suivantes :
- une valeur moyenne du ou des mesures environnementales comprises dans les données descriptives des épisodes événementiels sélectionnés relatifs à ce type d'événement environnemental ;
- une durée totale du déroulement de ce type d'événement environnemental ;
- le nombre d'épisodes événementiels sélectionnés de ce type d'événement environnemental ;
- des heures de début et de fin des épisodes événementiels sélectionnés de ce type d'événement environnemental.

6. Procédé selon l'une quelconque des revendications précédentes, **caractérisé en ce que** la période déterminée est définie automatiquement par l'unité de traitement en étant initié dès lors qu'un premier épisode événementiel d'un type d'événement environnemental est identifié, et achevée lorsqu'une durée d'exposition du porteur à au moins un type d'événement environnemental considéré comme perturbateur de la qualité de vie est supérieure à un seuil de durée de référence.

7. Système de diffusion (1) par une montre (100) d'un message informatif relatif à une évaluation de la qualité de vie d'un porteur de ladite montre (100) mettant en œuvre le procédé selon l'une quelconque des revendications précédentes, le système (1) comprenant les éléments suivants reliés entre eux : une unité de traitement (2), au moins un capteur environnemental (5) et une interface de diffusion d'une information visuelle et/ou sonore (3, 4).

8. Montre (100) comportant un système (1) selon la revendication précédente.

9. Montre (100) selon la revendication précédente, **caractérisée en ce qu'**elle est une montre (100) mécanique connectée.

10. Programme d'ordinateur comprenant des instructions de code de programme pour l'exécution des étapes (10 à 17) du procédé selon l'une quelconque des revendications 1 à 6 lorsque ledit programme est exécuté par une unité de traitement (2) d'un système de diffusion selon la revendication 7.

## Patentansprüche

1. Verfahren zum Verbreiten durch eine Uhr (100) einer informativen Nachricht, in Bezug auf eine Bewertung der Lebensqualität eines Trägers der Uhr (100), **dadurch gekennzeichnet, dass** das Verfahren die folgenden Schritte beinhaltet:
- Speichern (10) durch die Verarbeitungseinheit (2) von beschreibenden Daten von mindestens einer Ereignisepisode von mindestens einem Umweltereignistyp, die während eines bestimmten Zeitraums erhoben wurde;
- Identifizieren (11) von mindestens einem Umweltereignistyp, der die Lebensqualität des Trägers stört, anhand einer Verarbeitung der beschreibenden Daten;
- Schätzen (16) eines Bewertungsindex für die Lebensqualität des Trägers in Abhängigkeit von einem Störindikator der Lebensqualität in Bezug auf jeden identifizierten Typ eines die Lebensqualität störenden Umweltereignistyps, und
- Gestalten (17) der informativen Nachricht, die den geschätzten Bewertungsindex umfasst, zu deren Übermittlung an den Träger, und dadurch, dass der Schritt des Identifizierens (11) einen Teilschritt des Auswählens (12) einer oder mehrerer Ereignisepisoden in Bezug auf jeden störenden Umweltereignistyp für die Lebensqualität des Trägers, der während des anhand mindestens eines Auswahlkriteriums bestimmten Zeitraums erhoben worden ist, und dadurch, dass jede ausgewählte Ereignisepisode ein entsprechendes Umweltmaß aufweist, das größer als eine Bezugsschwelle für die Störung der Lebensqualität ist, und ein Maß für die entsprechende Dauer aufweist, das größer als eine Bezugsdauerschwelle ist.

2. Verfahren nach dem vorstehenden Anspruch, **dadurch gekennzeichnet, dass** ein erstes Auswahlkriterium vorsieht, dass mindestens ein Umweltmaß der beschreibenden Daten einer jeden Ereignisepisode in Bezug auf jeden Umweltereignistyp mit einer Referenzschwelle für die Störung der Lebensqualität verglichen wird.

3. Verfahren nach einem der Ansprüche 1 und 2, **dadurch gekennzeichnet, dass** ein zweites Auswahlkriterium darin besteht, eine Dauer jeder Ereignisepisode, die sich auf jeden Umweltereignistyp bezieht, mit einer Referenzschwelle für die Dauer der Störung der Lebensqualität zu vergleichen.

4. Verfahren nach einem der vorstehenden Ansprüche, **dadurch gekennzeichnet, dass** der Schritt des Identifizierens (11) einen Teilschritt des Erzeugens (14) eines Störindikators der Lebensqualität für jeden identifizierten Umweltereignistyp der die Lebensqualität stört, umfasst.

5. Verfahren nach dem vorstehenden Anspruch, **dadurch gekennzeichnet, dass** der Teilschritt des Erzeugens (14) eine Phase (15) zum Berechnen des Störindikators der Lebensqualität für jeden identifizierten Umweltereignistyp der die Lebensqualität stört, umfasst, der aus den folgenden beschreibenden Merkmalen identifiziert wird:
- einem Mittelwert des oder der Umweltmaß(e)s, der/die in den beschreibenden Daten der ausgewählten Ereignisepisoden in Bezug auf diesen Umweltereignistyp umfasst ist/sind;
- einer Gesamtdauer des Ablaufs dieses Umweltereignistyps;
- die Anzahl an ausgewählten Ereignisepisoden dieses Umweltereignistyps;
- der Anfangs- und Endzeit der ausgewählten Ereignisepisoden dieses Umweltereignistyps.

6. Verfahren nach einem der vorstehenden Ansprüche, **dadurch gekennzeichnet, dass** der bestimmte Zeitraum automatisch von der Verarbeitungseinheit als angestoßen definiert wird, sobald eine erste Ereignisepisode eines Umweltereignistyps identifiziert wird, und abgeschlossen wird, wenn eine Dauer der Aussetzung des Trägers gegenüber mindestens einem Umweltereignistyp, der als störend für die Lebensqualität betrachtet wird, größer ist als eine Referenzdauerschwelle.

7. System (1) zum Verbreiten durch eine Uhr (100) einer informativen Nachricht in Bezug auf eine Bewertung der Lebensqualität eines Trägers der Uhr (100), welches das Verfahren nach einem der vorstehenden Ansprüche umsetzt, wobei das System (1) die folgenden miteinander verbundenen Elemente umfasst: eine Verarbeitungseinheit (2), mindestens einen Umweltsensor (5) und eine Schnittstelle zur Verbreitung einer visuellen und/oder akustischen Information (3, 4).

8. Uhr (100), die ein System (1) nach dem vorstehenden Anspruch beinhaltet.

9. Uhr (100) nach dem vorstehenden Anspruch, **dadurch gekennzeichnet, dass** sie eine mechanische intelligente Uhr (100) ist.

10. Computerprogramm, das Programmcodeanweisungen zur Ausführung der Schritte (10 bis 17) des Verfahrens nach einem der Ansprüche 1 bis 6 umfasst, wenn das Programm von einer Verarbeitungseinheit (2) eines Systems zum Verbreiten (1) nach Anspruch 7 ausgeführt wird.

## Claims

1. Method for the broadcasting by a watch (100) of an informative message relating to an evaluation of the quality of life of a wearer of said watch (100), **characterised in that** the method comprises the following steps:
- recording (10), by the processing unit (2), of data describing at least one factual episode of at least one type of environmental event recorded during a given period;
- identification (11) of at least one type of environmental event disturbing the quality of life of the wearer from processing of said descriptive data;
- estimation (16) of an evaluation index of the quality of life of the wearer according to an indicator of disturbance of the quality of life relating to each type of environmental event disturbing the quality of life identified, and
- design (17) of the informative message comprising the estimated evaluation index in prediction of its broadcasting to the wearer, and wherein the identification step (11) comprises a substep (12) of selecting one or more factual episodes relating to each type of environmental event disturbing the quality of life of the wearer recorded during the given period using at least one selection criterion, and wherein each factual episode selected has a corresponding environmental measurement that is greater than a reference threshold of disturbance of the quality of life and has a corresponding duration measurement that is greater than a reference duration threshold.

2. Method according to the preceding claim, wherein a first selection criterion provides that at least one environmental measurement of the data describing each factual episode relating to each type of environmental event is compared with a reference threshold of disturbance of the quality of life.

3. Method according to any of claims 1 and 2, wherein a second selection criterion consists of comparing a duration of each factual episode relating to each type of environmental event with a reference threshold of duration of disturbance of the quality of life.

4. Method according to any of the preceding claims, wherein the identification step (11) comprises a substep (14) of generating an indicator of disturbance of the quality of life for each type of environmental event disturbing the quality of life identified.

5. Method according to the preceding claim, wherein the generation substep (14) comprises a phase (15) of calculating the indicator of disturbance of the quality of life for each type of environmental event disturbing the quality of life identified using the following descriptive characteristics:
- a mean value of the environmental measurement or measurements included in the data describing the selected factual episodes relating to this type of environmental event;
- a total duration of the occurrence of this type of environmental event;
- the number of selected factual episodes of this type of environmental event;
- times of start and end of the selected factual episodes of this type of environmental event.

6. Method according to any of the preceding claims, wherein the given period is defined automatically by the processing unit by being initiated as soon as a first factual episode of a type of environmental event is identified, and by being completed when a duration of exposure of the wearer to at least one type of environmental event considered to be disturbing the quality of life is higher than a reference duration threshold.

7. System (1) for the broadcasting, by a watch (100), of an informative message relating to an evaluation of the quality of life of a wearer of said watch (100) using the method according to any of the preceding claims, the system (1) comprising the following elements connected together: a processing unit (2), at least one environmental sensor (5) and an interface broadcasting visual and/or audible information (3, 4).

8. Watch (100) comprising a system (1) according to the preceding claim.

9. Watch (100) according to the preceding claim, wherein it is a connected mechanical watch (100).

10. Computer program comprising program code instructions for executing the steps (10 to 17) of the method according to any of claims 1 to 6 when said program is executed by a processing unit (2) of a transmission system (1) according to claim 7.
